# EUROPEAN PATENT APPLICATION

(11) **EP 3 078 382 A1**
(43) Date of publication of application: **12.10.2016**
(21) Application number: 15722345.4
(22) Date of filing: 10.03.2015
(51) Int. Cl.: A61K 36/8945, A61K 36/428, A61K 36/48, A61K 36/489, A61K 36/70, A61P 3/10, A61P 3/06, A61P 1/16

(54) **MEDICINE FOR PROTECTING THE LIVER, REMOVING TOXINS, REDUCING BLOOD LIPIDS AND REDUCING BLOOD SUGAR AND USE THEREOF**

(30) Priority: 12.02.2015 CN 201510756121
(71) Applicant: Zhao, Junhong, JiuLongpo District Chongqing (CN)
(72) Inventor: Zhao, Junhong, JiuLongpo District Chongqing (CN)
(74) Representative: Hautier IP
(86) International application number: PCT/CN2015/073924
(87) International publication number: WO 2016/127463

(57) **Abstract**

The present invention provides a drug showing liver protection, detoxification, lipid-lowering and hypoglycemic properties and use thereof, which is a formulation prepared from a starting material comprising, in parts by weight, 10-35 parts of the root of Trichosanthes cucumeroides (Ser.) Maxim, 5-20 parts of the root of Radix Sophorae Flavescentis, 15-30 parts of Tartary Buckwheat, 15-25 parts of the root of Rhizoma Dioscorea, 10-35 parts of the root of Kudzu Vine and 10-25 parts of pollen. It is useful in the manufacturing of a medicament for treating liver diseases, in the manufacturing of a medicament for treating hyperlipidemia or in the manufacturing of a medicament for treating diabetes. The drug of the present invention takes advantages of the medicinal properties of the root of Trichosanthes cucumeroides (Ser.) Maxim and the root of Radix Sophorae Flavescentis, to achieve liver-clearing and cholagogue effects, and a therapeutical effect on jaundice and chronic hepatitis, in combination with the spleen-nourishing, Qi-regulating, pathogenic wind-dispelling and phlegm-eliminating properties of Tartary Buckwheat, the spleen-nourishing, appetizing, deficiency restoring properties of the root of Rhizoma Dioscorea, the deficient heat clearing and alcoholism relieving properties of the root of Kudzu Vine, and the thirst relieving and jaundice clearing properties of pollen. Thus, liver protection, detoxification, lipid-lowering and hypoglycemic effects can be achieved by the drug of the present invention. The drug of the present invention further provides the following advantages: its raw materials can be obtained from abundant sources; its formulation can be prepared using conventional processes, through simple procedures, and at a low cost; it is much worthy of promotion and application, and has a promising market prospect and a vast space for development.

## Description

### Technical Field

The present invention relates to the field of traditional Chinese medicine composition, in particular to a drug showing liver protection, detoxification, lipid-lowering and hypoglycemic properties and use thereof

### Background Art

Hepatitis is an inflammation of the liver, which may have a lot of complications caused by the abnormality in the hematopoietic function, and consequently will do serious harm to the health of human beings. Due to the variety of types of hepatitis and the complexity of pathogenic causes thereof, if it is not treated in time or in an effective way, its condition will change with time. Thus, it is important to protect the liver. According to incomplete statistics, currently in China, there are about one hundred million people infected with hepatitis B virus, and 20 million people suffered from chronic hepatitis B, which makes China a veritable "large HBV country". More than 80% of patients in China having serious diseases, such as cirrhosis, liver cancer and the like, are originated from patients suffered from Hepatitis B. It is thus clear that, if the effectiveness of a drug is verified during the research, it would have a considerable prospect in development. Currently, the treatment of hepatitis, especially the treatment of hepatitis B, carried out in medical practice is mainly focused on anti-viral therapy. Since antiviral therapy has specific indications and contraindications, and is limited by various factors including, among other, economical conditions, patients that can receive regular anti-viral therapy are relatively limited, most patients can only receive anti-inflammatory and liver protection treatment to prevent further progression of their diseases.

Furthermore, chronic diseases including hyperlipidemia, diabetes and the like become diseases endangering the health or even lives of human beings, which makes most patients suffered from such diseases have to take medicines to control the situation. This may cause a great deal of financial stress to the patients, as those medicines are generally high in cost.

Consequently, there is an urgent need to develop a drug for treating chronic diseases such as hepatitis, hyperlipidemia, diabetes, and the like, which has a wide applicability and a relatively reasonable cost.

### Summary of the Invention

In view of the above deficiencies and problems present in the prior arts, an object of the present invention is to provide a drug showing liver protection, detoxification, lipid-lowering and hypoglycemic properties and use thereof, which allows the achievement of the effects of recovering liver diseases in early and mid stages, lowering the blood lipids, lowering the blood sugar, reducing body weight and shaping the body, relaxing the bowels, and promoting excretion (both defecation and urination). Meanwhile, the drug of the present invention is simple in preparation and its raw materials are abundant in sources, so that the problems associated with the high price and poor efficacy of medicines currently used in the treatment of hepatitis, hyperlipidemia and diabetes can be solved.

To accomplish the above object, the present invention provides the following technical solution: a drug showing liver protection, detoxification, lipid-lowering and hypoglycemic properties, which is a formulation prepared from a starting material comprising, in parts by weight, 10-35 parts of the root of Trichosanthes cucumeroides (Ser.) Maxim, 5-20 parts of the root of Radix Sophorae Flavescentis, 15-40 parts of Tartary Buckwheat, 5-25 parts of the root of Rhizoma Dioscorea, 10-40 parts of the root of Kudzu Vine and 10-30 parts of pollen.

Preferably, the drug is a formulation prepared from a starting material comprising, in parts by weight, 10-20 parts of the root of Trichosanthes cucumeroides (Ser.) Maxim, 5-15 parts of the root of Radix Sophorae Flavescentis, 20-30 parts of Tartary Buckwheat, 15-20 parts of the root of Rhizoma Dioscorea, 10-30 parts of the root of Kudzu Vine and 15-20 parts of pollen.

Preferably, the drug is a formulation prepared from a starting material comprising, in parts by weight, 15 parts of the root of Trichosanthes cucumeroides (Ser.) Maxim, 15 parts of the root of Radix Sophorae Flavescentis, 30 parts of Tartary Buckwheat, 15 parts of the root of Rhizoma Dioscorea, 10 parts of the root of Kudzu Vine and 20 parts of pollen.

Preferably, the drug is a formulation prepared from a starting material comprising, in parts by weight, 15 parts of the root of Trichosanthes cucumeroides (Ser.) Maxim, 20 parts of the root of Radix Sophorae Flavescentis, 30 parts of Tartary Buckwheat, 15 parts of the root of Rhizoma Dioscorea, 10 parts of the root of Kudzu Vine and 20 parts of pollen.

Preferably, the drug is a formulation prepared from a starting material comprising, in parts by weight, 10 parts of the root of Trichosanthes cucumeroides (Ser.) Maxim, 10 parts of the root of Radix Sophorae Flavescentis, 20 parts of Tartary Buckwheat, 15 parts of the root of Rhizoma Dioscorea, 30 parts of the root of Kudzu Vine and 25 parts of pollen.

Preferably, the drug is a formulation prepared from a starting material comprising, in parts by weight, 10 parts of the root of Trichosanthes cucumeroides (Ser.) Maxim, 5 parts of the root of Radix Sophorae Flavescentis, 30 parts of Tartary Buckwheat, 25 parts of the root of Rhizoma Dioscorea, 10 parts of the root of Kudzu Vine and 25 parts of pollen.

Preferably, the formulation is in an oral dosage form, such as tablets, capsules, electuaries, granules, powders, or the like, commonly used in medical practice.

The drug showing liver protection, detoxification, lipid-lowering and hypoglycemic properties according to the present invention can be prepared using traditional Chinese medicine processing methods and processes known in the art.

The use of the drug showing liver protection, detoxification, lipid-lowering and hypoglycemic properties according to the present invention in the manufacturing of a medicament for treating liver diseases is also provided herein.

The use of the drug showing liver protection, detoxification, lipid-lowering and hypoglycemic properties according to the present invention in the manufacturing of a medicament for treating hyperlipidemia is also provided herein.

The use of the drug showing liver protection, detoxification, lipid-lowering and hypoglycemic properties according to the present invention in the manufacturing of a medicament for treating diabetes is also provided herein.

The drug showing liver protection, detoxification, lipid-lowering and hypoglycemic properties according to the present invention is formulated based on the knowledge as described below.

The root of Trichosanthes cucumeroides (Ser.) Maxim is bitter in taste, cold in nature, and distributed to liver, spleen, and stomach channels. The root of Trichosanthes cucumeroides (Ser.) Maxim is rich in fatty acids and further contains kaempferitrin, amino acids, carotene, choline and the like. It has detoxification, anticancer, heat purging, stagnated blood removing, detumescence, analgesia, ulcerative carbuncle curing and pyogenic infections relieving properties.

The root of Radix Sophorae Flavescentis is bitter in taste, cold in nature, and distributed to liver, kidney, colon, small intestine channel, bladder, heart channel and bladder channel. It has down drawing, heat clearing, damp drying, damp clearing, jaundice removing, pathogenic wind dispelling and insecticidal properties, and can achieve anti-tumor, leukocyte raising, cardiovascular system improving, antiasthmatic, expectorant, stabilizing, anti-allergic, immunosuppressione effects and the like.

Tartary Buckwheat, as described in "Compendium of Materia Medica", is bitter in taste while slightly cold in nature, and has the properties of improving intestines and stomach, raising strength and energy, keeping the spirit, improving ears and eyes, converting contaminants in the five internal organs. Meanwhile, in "Invaluable Prescriptions for Ready Reference", "The Dictionary of Medicinal Plant" and other related documents, Tartary Buckwheat is described to have the effects of soothing the nerves, promoting blood circulations, depressing Qi and relaxing intestines, clearing heat swelling and rheumatic arthralgia, relieving dyspepsia, purging gut, moistening the intestines, defaecating, relieving cough and asthma, anti-inflammatory, anti-allergy, strengthening the heart, reducing body weight, improving the beauty, and the like. And, it is a crop that is not only edible but also useful in drugs.

The root of Rhizoma Dioscorea is sweet in taste, neutral in nature, and distributed to spleen, lung and kidney channels. It has the properties of invigorating spleen, nourishing stomach, generating saliva, benefiting lung, tonifying kidney and arresting seminal emission.

The root of Kudzu Vine is cool in nature, while sweet and acrid in taste. It is normally used to treat the diseases including typhoid fever, headache and cervical rigidity (stiff neck), thirst in vexed heat, diarrhea, measles without adequate eruption, hypertension, angina, epicophosis and the like.

Pollen contains a lot of nutrients and physiologically active substances, such as starch, glucose, fructose, vitamins, enzymes and the like. It has the properties of regulating the nervous system, promoting sleep, enhancing physical fitness; improving gastrointestinal function, promoting digestion, increasing appetite, preventing habitual constipation, hepatoprotection, prevention and treatment of anemia, diabetes and the like; endocrine regulation, improving body functions, and the like.

The drug showing liver protection, detoxification, lipid-lowering and hypoglycemic properties according to the present invention has a reasonable prescription, with all raw materials thereof being used in an amount within an appropriate range, and thus is non-toxic. The drug of the present invention takes advantages of the medicinal properties of the root of Trichosanthes cucumeroides (Ser.) Maxim and the root of Radix Sophorae Flavescentis, to achieve liver-clearing, cholagogue, damp drying, and jaundice removing effects, as well as a therapeutical effect on chronic hepatitis, in combination with the spleen-nourishing, Qi-regulating, pathogenic wind-dispelling and phlegm-eliminating properties of Tartary Buckwheat, the spleen-nourishing, appetizing, deficiency restoring properties of the root of Rhizoma Dioscorea, the deficient heat clearing and alcoholism relieving properties of the root of Kudzu Vine, and the thirst relieving and jaundice clearing properties of pollen. Thus, the drug of the present invention has liver protection, detoxification, lipid-lowering and hypoglycemic properties, and can achieve the effects of detoxifying liver and treating fatty liver disease, alcohol liver disease, liver cirrhosis, hepatitis and liver ascites. The inventive drug also exhibits a special therapy effect on constipation, so that patients with constipation can be completely cured after receiving a 15 days treatment with said drug via oral administration. The inventive drug can also be used for the treatment of toothache, with a special efficacy of relieving the toothache quickly in a few hours and making a loose tooth to restore its original position spontaneously in three days and recover steadily. Where the inventive drug is used for lowering the blood sugar and the urine sugar of patients, insulin injection to the patients can be cancelled in a few days, complications can be stopped, and most patients will be rehabilitated in 3-4 months.

The drug of the present invention further provides the following advantages: its raw materials can be obtained from abundant sources; its formulation can be prepared using conventional processes, through simple procedures, and at a low cost; it is much worthy of promotion and application, and has a promising market prospect and a vast space for development.

### Detailed Description of the Invention

Hereinafter, the subject matter of the present invention will be illustrated in a clear and complete manner with reference to the following examples. It is obvious that the examples as described below represent not all but only a part of the embodiments according to the present invention. All embodiments that can be obtained, based on the examples described herein, by those skilled in the art without doing any inventive work shall be deemed to fall within the scope of the present invention.

### Example 1

This example provided a drug showing liver protection, detoxification, lipid-lowering and hypoglycemic properties, which was in the form of a capsule formulation prepared from a starting material comprising, in parts by weight, 15 parts of the root of Trichosanthes cucumeroides (Ser.) Maxim, 15 parts of the root of Radix Sophorae Flavescentis, 30 parts of Tartary Buckwheat, 15 parts of the root of Rhizoma Dioscorea, 10 parts of the root of Kudzu Vine and 20 parts of pollen.

In Example 1, the drug showing liver protection, detoxification, lipid-lowering and hypoglycemic properties according to the present invention was prepared into a capsule formulation using a conventional process for preparing capsules. In particular, the formulation was prepared according to the following procedures: each raw material was weighted in parts as described about, exacted separately with an alcohol and then ground into powders; all powders were mixed together to yield a mixture of raw materials; an excipient or auxiliary ingredient commonly used in medicines was added into the mixture of raw materials to obtain a pharmaceutical mixture; and then the pharmaceutical mixture was filled into capsules.

The capsules filled with the drug of this example showing liver protection, detoxification, lipid-lowering and hypoglycemic properties were 0# capsules, with each capsule comprising a controlled effective dose (i.e. the amount of the mixture of raw materials) of 0.38g.

In Example 1, the drug of showing liver protection, detoxification, lipid-lowering and hypoglycemic properties was used to prepare a medicament for the treatment of liver diseases.

The effect of the medicament for the treatment of liver diseases prepared from the inventive drug was tested in the following clinical cases.

Dosage schedule: 1 to 2 times per day, one capsule per time.

Mr. Qin was a 38 years old patient suffered from hepatitis B. The measured value of alanine aminotransferase for this patient was 456 before medication and 16 after medication, while the measured value of aspartate aminotransferase was 257 before medication and 26 after medication, with the duration of medication being 60 days. Test results showed that the liver function of the patient had returned to normal.

Mr. Xie was a 45 years old patient suffered from fatty liver and alcoholic liver diseases. After a continuous medication for 60 days with the inventive drug, those diseases of the patient were both cured.

### Example 2

This example provided a drug showing liver protection, detoxification, lipid-lowering and hypoglycemic properties, which was in the form of a tablet formulation prepared from a starting material comprising, in parts by weight, 15 parts of the root of Trichosanthes cucumeroides (Ser.) Maxim, 20 parts of the root of Radix Sophorae Flavescentis, 30 parts of Tartary Buckwheat, 15 parts of the root of Rhizoma Dioscorea, 10 parts of the root of Kudzu Vine and 20 parts of pollen.

The tablet formulation of Example 2 was prepared according to the following procedures: each raw material was weighted in parts as described about, exacted separately with an alcohol and then ground into powders; all powders were mixed together to yield a mixture of raw materials; an excipient or auxiliary ingredient commonly used in medicines was added into the mixture of raw materials to obtain a pharmaceutical mixture; and then the pharmaceutical mixture was formed into tablets using a conventional extrusion process for preparing tablets. Each tablet had a weight of 1 gram, and an effective dose (i.e. the amount of the mixture of raw materials) of 0.35g.

In Example 2, the drug of showing liver protection, detoxification, lipid-lowering and hypoglycemic properties was used to prepare a medicament for the treatment of proteinuria.

The effect of the medicament for the treatment of proteinuria prepared from the inventive drug was tested in the following clinical cases.

Dosage schedule: 1 to 2 times per day, one table (i.e. 1 g) of the medicament of Example 2 per time.

Mr. Long was a 15 years old patient suffered from proteinuria. Before medication, the measured value of protein concentration (i.e. PRO) for this patient was 533.6, while the measured value of microalbuminuria (i.e. MAL) was 361. After a medication for 10 days with the inventive drug, each indicator observed in a recheck received by the patient had returned to normal. A recheck was further performed on the patient at 5 months after the end of the medication, in which each indicator observed remained at a normal level.

### Example 3

This example provided a drug showing liver protection, detoxification, lipid-lowering and hypoglycemic properties, which was in the form of a granule formulation prepared from a starting material comprising, in parts by weight, 10 parts of the root of Trichosanthes cucumeroides (Ser.) Maxim, 10 parts of the root of Radix Sophorae Flavescentis, 20 parts of Tartary Buckwheat, 10 parts of the root of Rhizoma Dioscorea, 40 parts of the root of Kudzu Vine and 10 parts of pollen.

The formulation of the drug of Example 3 showing liver protection, detoxification, lipid-lowering and hypoglycemic properties was prepared according to the process as described in Example 2, except that the pharmaceutical mixture obtained was formed into granules using a conventional process for preparing granules. Each granule had a controlled weight of 5g, and an effective dose (i.e. the amount of the mixture of raw materials) of 0.5g.

In Example 3, the drug of showing liver protection, detoxification, lipid-lowering and hypoglycemic properties was used to prepare a medicament for the treatment of diabetes.

The effect of the medicament for the treatment of diabetes prepared from the inventive drug was tested in the following clinical cases.

Dosage schedule: 1 to 2 times per day for 60 days, one granule (i.e. 5 g) of the granule formulation of Example 3 per time.

Mr. Liu was a 44 years old patient suffered from diabetes for 5 years, and had complications of lacrimation, pedal flaccidity and dizziness. After a medication for 5 days with the medicament of this example, the administration of other hypoglycemic drugs was cancelled; after a continuous medication for 10 days with the medicament of this example, those complications of lacrimation, pedal flaccidity and dizziness were significantly relieved; and after a continuous medication for 2 months, the measured value of fasting blood glucose for the patient was between 6.5 and 6.8.

Mr. Du was a 76 years old patient suffered from diabetes, with the measured value of fasting blood glucose being 12.9, and had complications of gout, as well as pain and numbness in right leg. After a medication for 3 days with the medicament of this example, the insulin injection was cancelled; after a continuous medication for 10 days with the medicament of this example, the complications of gout did not attack, and the symptom of pain and numbness in the leg disappeared; and after a continuous medication for 30 days and a discontinuation period thereafter of 10 days, the measured value of fasting blood glucose for the patient was 7.6.

The "alcohol extraction" step mentioned in Examples 1-3 of the present application was performed using a conventional process.

### Example 4

This example provided a drug showing liver protection, detoxification, lipid-lowering and hypoglycemic properties, which was in the form of a decoction formulation prepared from a starting material comprising, in parts by weight, 10 parts of powders of the root of Trichosanthes cucumeroides (Ser.) Maxim, 5 parts of the root of Radix Sophorae Flavescentis, 30 parts of Tartary Buckwheat, 25 parts of the root of Rhizoma Dioscorea, 10 parts of the root of Kudzu Vine and 25 parts of pollen.

The formulation was prepared according to the following procedures: each raw material was weighted in parts as described about, exacted separately with water or an organic solvent, the extracted active ingredients were mixed together, and then an excipient or auxiliary ingredient (eg. honey) commonly used in medicines was added into the resulting mixture to yield the decoction. The decoction had a controlled effective dose of 0.5g per 20 grams of the decoction.

In Example 4, the drug of showing liver protection, detoxification, lipid-lowering and hypoglycemic properties was used to prepare a medicament for the treatment of constipation.

The effect of the medicament for the treatment of constipation prepared from the inventive drug was tested in the following clinical cases.

Dosage schedule: 1 to 2 times per day for 10 days, 20 grams of the decoction formulation of Example 4 per time.

Ms. Chen was a 58 years old patient suffered from constipation, who defecated once every five days and needed to use a compound to help the defecation. One day after taking the medicament of this example, the patient defecated twice; after a continuous medication for 15 days, the symptoms of constipation of the patient disappeared; and after the medication was stopped, the defecation of the patient remained once per day and the sleeping quality of the patient was significantly improved.

### Example 5

This example provided a drug showing liver protection, detoxification, lipid-lowering and hypoglycemic properties, which was in the form of a powders formulation prepared from a starting material comprising, in parts by weight, 10 parts of the root of Trichosanthes cucumeroides (Ser.) Maxim, 5 parts of the root of Radix Sophorae Flavescentis, 40 parts of Tartary Buckwheat, 5 parts of the root of Rhizoma Dioscorea, 10 parts of the root of Kudzu Vine and 30 parts of pollen.

The formulation was prepared according to the following procedures: each raw material was weighted in parts as described about, ground separately into powders, mixed together to obtain a mixture of the raw materials, and then the resulting mixture was filled into packets with each packet containing 2 grams of powders. Each raw material used in this example was in the form of blocks or granules.

The effect of the inventive drug in the treatment of hyperlipidemia and in shaping the body and reducing body weight was tested in the following clinical cases.

Dosage schedule: 1 to 2 times per day for 40 days, one packet (2g) per time.

Mr. Fang was a 45 years old man, who was 1.6 meter high while had a body weight of 82.5 Kg. After a medication for 40 days with the drug of this example, his body weight was reduced to 67 Kg, and he was full of energy and good in physical condition. Meanwhile, the measured results of his blood lipids and blood pressure were at a normal level.

Besides the oral dosage form as described above, the drug showing liver protection, detoxification, lipid-lowering and hypoglycemic properties according to the present invention can also be prepared into other dosages forms, including pills, oral solutions, and the like. All dosage forms of the present invention can be prepared using traditional Chinese medicine processing methods.

The key to the present invention is the appropriate combination and amount used of the raw materials, which allows the achievement of the special properties and efficacy of the inventive drug.

The specific embodiments as described above only represent some of the embodiments of the present invention, and shall not be interpreted as a limitation to the scope of the present invention. Various modifications and variations of those embodiments can be readily obtained by those skilled in the art without departing from the spirit of the present invention, and thus shall be deemed to fall within the scope of the present invention. Accordingly, the scope of the present invention shall only be defined in the appended claims.

## Claims

1. A drug showing liver protection, detoxification, lipid-lowering and hypoglycemic properties, **characterized in that**, said drug is in the form of a formulation prepared from a starting material comprising, in parts by weight, 10-35 parts of the root of Trichosanthes cucumeroides (Ser.) Maxim, 5-20 parts of the root of Radix Sophorae Flavescentis, 15-40 parts of Tartary Buckwheat, 5-25 parts of the root of Rhizoma Dioscorea, 10-40 parts of the root of Kudzu Vine and 10-30 parts of pollen.

2. The drug showing liver protection, detoxification, lipid-lowering and hypoglycemic properties according to claim 1, **characterized in that**, it is in the form of a formulation prepared from a starting material comprising, in parts by weight, 10-20 parts of the root of Trichosanthes cucumeroides (Ser.) Maxim, 5-15 parts of the root of Radix Sophorae Flavescentis, 20-30 parts of Tartary Buckwheat, 15-20 parts of the root of Rhizoma Dioscorea, 10-30 parts of the root of Kudzu Vine and 15-20 parts of pollen.

3. The drug showing liver protection, detoxification, lipid-lowering and hypoglycemic properties according to claim 1, **characterized in that**, it is in the form of a formulation prepared from a starting material comprising, in parts by weight, 15 parts of the root of Trichosanthes cucumeroides (Ser.) Maxim, 15 parts of the root of Radix Sophorae Flavescentis, 30 parts of Tartary Buckwheat, 15 parts of the root of Rhizoma Dioscorea, 10 parts of the root of Kudzu Vine and 20 parts of pollen.

4. The drug showing liver protection, detoxification, lipid-lowering and hypoglycemic properties according to claim 1, **characterized in that**, it is in the form of a formulation prepared from a starting material comprising, in parts by weight, 15 parts of the root of Trichosanthes cucumeroides (Ser.) Maxim, 20 parts of the root of Radix Sophorae Flavescentis, 30 parts of Tartary Buckwheat, 15 parts of the root of Rhizoma Dioscorea, 10 parts of the root of Kudzu Vine and 20 parts of pollen.

5. The drug showing liver protection, detoxification, lipid-lowering and hypoglycemic properties according to claim 1, **characterized in that**, it is in the form of a formulation prepared from a starting material comprising, in parts by weight, 10 parts of the root of Trichosanthes cucumeroides (Ser.) Maxim, 10 parts of the root of Radix Sophorae Flavescentis, 20 parts of Tartary Buckwheat, 10 parts of the root of Rhizoma Dioscorea, 40 parts of the root of Kudzu Vine and 10 parts of pollen.

6. The drug showing liver protection, detoxification, lipid-lowering and hypoglycemic properties according to claim 1, **characterized in that**, it is in the form of a formulation prepared from a starting material comprising, in parts by weight, 10 parts of the root of Trichosanthes cucumeroides (Ser.) Maxim, 5 parts of the root of Radix Sophorae Flavescentis, 30 parts of Tartary Buckwheat, 25 parts of the root of Rhizoma Dioscorea, 10 parts of the root of Kudzu Vine and 25 parts of pollen.

7. The drug showing liver protection, detoxification, lipid-lowering and hypoglycemic properties according to one of claims 1-6, **characterized in that**, the formulation is in an oral dosage form commonly used in medical practice.

8. Use of a drug showing liver protection, detoxification, lipid-lowering and hypoglycemic properties according to one of claims 1-6 in the manufacturing of a medicament for treating liver diseases.

9. Use of a drug showing liver protection, detoxification, lipid-lowering and hypoglycemic properties according to one of claims 1-6 in the manufacturing of a medicament for treating hyperlipidemia.

10. Use of a drug showing liver protection, detoxification, lipid-lowering and hypoglycemic properties according to one of claims 1-6 in the manufacturing of a medicament for treating diabetes.
